# EUROPÄISCHE PATENTSCHRIFT

(11) **EP 3 970 512 B1**
(45) Veröffentlichungstag und Bekanntmachung des Hinweises auf die Patenterteilung: **01.11.2023**
(21) Anmeldenummer: 21196774.0
(22) Anmeldetag: 15.09.2021
(51) Int. Cl.: A23L 33/135, A23L 29/00, A23L 19/00, A61K 35/745, A61K 35/747, A61K 36/22

(54) **VERFAHREN ZUR HERSTELLUNG EINER PROBIOTISCHEN FRUCHTKOMPOSITION SOWIE PROBIOTISCHE FRUCHTKOMPOSITION**
PROBIOTIC FRUIT COMPOSITION AND METHOD OF PREPARING A PROBIOTIC FRUIT COMPOSITION
PROCÉDÉ DE PRODUCTION D'UNE COMPOSITION PROBIOTIQUE À BASE DE FRUITS ET COMPOSITION PROBIOTIQUE À BASE DE FRUITS

(30) Priorität: 16.09.2020 DE 102020124073
(43) Veröffentlichungstag der Anmeldung: 23.03.2022
(73) Patentinhaber: Döhler GmbH, 64295 Darmstadt (DE)
(72) Erfinder: Penning, Björn, 64283 Darmstadt (DE); Bick, Annette, 21355 Lüneburg (DE); Foltz, Martin, 64625 Bensheim (DE); Reeg, Laura, 64347 Griesheim (DE); Smerz, Alexander, 64295 Darmstadt (DE)
(74) Vertreter: Tesch, Sabine

(56) Entgegenhaltungen:
- EP-A1- 2 168 441
- CN-A- 106 605 919
- CN-A- 108 013 473
- CN-A- 109 463 673

## Beschreibung

Die vorliegende Erfindung betrifft ein Verfahren zur Herstellung einer probiotischen Fruchtkomposition sowie eine probiotische Fruchtkomposition.

Die Internationale Wissenschaftliche Vereinigung für Probiotika und Präbiotika (ISAPP) hat die Definition von Probiotika der Food and Agriculture Organisation FAO der Vereinten Nationen beziehungsweise der World Health Organisation WHO ratifiziert: Probiotika sind demnach "lebende Mikroorganismen, die, wenn sie in ausreichenden Mengen verabreicht werden, dem Wirt einen gesundheitlichen Nutzen verschaffen". Typische probiotische Mikroorganismen sind Bakterienstämme, die zur Gattung *Lactobacillus, Bifidobacterium, Enterococcus, Lactococcus, Propionibacterium, Bacillus, Streptococcusoder Escherichia* gehören. Heutzutage werden Probiotika hauptsächlich in Milchprodukten, milchähnlichen Produkten sowie in Nahrungsergänzungsmitteln wegen ihrer anspruchsvollen Überlebensbedingungen, kurzen Haltbarkeit und ihres Einflusses auf die sensorischen Eigenschaften eines Lebensmittelproduktes eingesetzt.

Ein wesentliches Merkmal der sogenannten "probiotischen" Lebensmittel ist der Gehalt an lebensfähigen Zellen von probiotischen Mikroorganismen. Nur durch eine entsprechende Anzahl von probiotischen Mikroorganismen pro Verzehreinheit wird ein positiver Einfluss, beispielsweise auf die Darmflora, erreicht. Der sogenannte "minimale therapeutische" Gehalt an lebensfähigen probiotischen Mikroorganismen soll mindestens 10⁶ KBE (Koloniebildende Einheiten) in einer Verzehreinheit (bzw. in einer Portion eines Lebensmittels) während der ganzen Produkthaltbarkeit des probiotischen Lebensmittels sein.

Bisher wurden Probiotika als Nahrungsergänzungsmittel in trockenem Zustand in Form von z.B. Pulvern, Kapseln usw. verwendet oder einem gekühlten flüssigen Lebensmittel mit einer kurzen Haltbarkeitsdauer zugegeben. Zum Beispiel ist aus EP 0 818 529 B1 ein Trocknungsverfahren zur Pulverherstellung bekannt. WO 2010/054439 A1 offenbart eine probiotische Komposition, in der Probiotika in einer Matrix aus Kohlenhydraten, Milchprodukten, Zucker, Siliciumdioxid, Stärke usw. eingeschlossen sind.

Bisher wurde die meiste Arbeit bezüglich Stabilisierung und Überlebensfähigkeit von Probiotika in Nahrungsmitteln auf Basis von Milchprodukten durchgeführt. In der letzten Zeit hat die Forschung mit der Verwendung von Probiotika in Nicht-Milchprodukten angefangen. EP 1 482 811 B1 offenbart beispielsweise ein Verfahren zur Herstellung von beschichteten Pellets (Pellets für Tierfutter), die lebensfähige Mikroorganismen enthalten.

Aktuell wurden folgende Herangehensweisen entwickelt, um den Einsatz von Probiotika vor allem in nicht gekühlten Lebensmitteln zu ermöglichen:
- Einsatz von probiotischen Bakterien, die Sporen bilden können, in Lebensmittelanwendungen. Vorteil dieser Methode ist die extreme Beständigkeit der Sporen gegen Hitze, Säure, Druck und hohe Wasseraktivitätswerte (a_{w}). Dazu werden die probiotischen Bakterien in ihrer Sporenform eingesetzt. Allerdings zählen zur Klasse der sporenbildenden Bakterien hauptsächlich Verderber von Nahrungsmitteln, die sowohl die Produktqualität als auch die Produktsicherheit stark negativ beeinflussen können. Da die in der Nahrungsmittelproduktion eingesetzten Zutaten und Zusatzstoffe in der Regel frei von sporenbildenden Bakterien sein müssen, lehnt die Lebensmittelindustrie aufgrund erhöhter Reinigungs- und Hygieneaufwendungen probiotische sporenbildende Bakterien weitgehend ab.
- Verkapselung der Mikroorganismen. Diese wird regelmäßig verwendet, um die Überlebensfähigkeit von Probiotika, insbesondere während der Magen-Darm-Passage, zu verbessern. In letzter Zeit wurden einige Versuche unternommen, verkapselte Mikroorganismen-Stämme zwecks Stabilitätserhöhung gegenüber Einflüssen aus dem Lebensmittel wie Druck, Temperatur und Wassergehalt sowie während der Magen-Darm-Passage in einer Lebensmittelmatrix einzusetzen. Verschiedene Materialien, wie Polysaccharide, Proteine oder Fette, werden für die Verkapselung von probiotischen Zellen getestet und zum Teil eingesetzt.

CN 108 013 473 A beschreibt gefriergetrocknete probiotische Partikel und verzehrfertigen Haferbrei mit Obst mit dem Ziel, probiotische Produkte für den Markt der Cerealien bereitzustellen. Es werden gefriergetrocknete Partikel aus Maltodextrin, Zucker, Pektin, Probiotika und Obst- und Gemüsesaftkonzentrat hergestellt und im Endprodukt gefriergetrocknete oder kandierte Früchte eingesetzt.

CN 109 463 673 A beschreibt ein Verfahren zum Herstellen gefriergetrockneter Obst- und Gemüsepartikeln durch probiotische Fermentation. Es werden vorbereitete Fruchtstücke mit Maltodextrin und modifizierter Stärke versetzt und mit einer Fermentationslösung aus entfärbtem Fruchtsaft und Oligofructose gemischt. Das sterilisierte Material wird mit Probiotika geimpft, fermentiert und anschließend gefriergetrocknet.

CN 106 605 919 A beschreibt eine Zusammensetzung zur Verwendung gegen Verdauungsstörungen bei Kindern, die Obst- und Gemüsepulver und Probiotika neben anderen Bestandteilen enthält. Die Probiotika liegen in gefriergetrockneter Form und insbesondere als Pulver in der Mischung vor. Die Komponenten des Obst- und Gemüsepulvers sowie das gefriergetrocknete Probiotika-Pulver werden gemischt und dann abgefüllt.

EP 2 168 441 A1 beschreibt ein Lebensmittel enthaltend mindestens 95 Gew.-% Obst- und/oder Gemüsezubereitungen mit probiotischen Keimen neben weiteren Inhaltsstoffen. Typische probiotische Mikroorganismen sind demnach Milchsäurebakterien. Das Lebensmittel setzt sich aus den üblichen Zubereitungen für Smoothies nebst Zusätzen zusammen.

Es besteht weiterhin Bedarf an anderen Lebensmitteln, die sich nicht nur durch gute Lagerungseigenschaften und hohe Gehalte an probiotischen Mikroorganismen auszeichnen, sondern auch einen verbesserten Geschmack, eine attraktivere Farbe und eine bessere Handhabung im Vergleich zu den handelsüblichen Probiotika-Produkten, insbesondere auf der Basis von Milchprodukten, aufweisen. Als Nahrungsergänzungsmittel sind lyophyliserte Probiotika "as is" in Pulverform handelsüblich. Diese sind sensorisch suboptimal, da sie oft leicht bitter schmecken und ein sandiges Mundgefühl verursachen.

Trockene Früchte bzw. trockene fruchthaltige Produkte sind durch ihre höhere Nährstoffkonzentration für viele Menschen geschmacklich attraktiv und liefern mehr Vitamine und Mineralstoffe als die gleiche Menge frischer Früchte, jedoch stellt eine Anreicherung von lebensfähigen probiotischen Mikroorganismen hohe Anforderungen an Lebensmittel, insbesondere an fruchthaltige Lebensmittel.

Probiotische Bakterien sind einerseits sehr hitzeempfindlich, d.h. es gibt viele Einschränkungen, die die Verwendung von verschiedenen Trocknungsmethoden betreffen. Ein zu starkes Absterben der probiotischen Bakterien während der Lebensmittelproduktion und über die Lagerungsdauer stellt die probiotische Wirksamkeit des Produktes in Frage. Anderseits stellen Probiotika lebendige, vermehrungsfähige Bakterien dar, die in der Lage sind, den Zustand eines Lebensmittels negativ zu beeinflussen, wenn die Lebensmittelmatrix das Wachstum der probiotischen Bakterien fördert. Ein unkontrolliertes Wachstum kann zu Geschmacks- oder Geruchsfehlnoten führen, somit die Sensorik des Lebensmittel beeinflussen und wäre ebenfalls inakzeptabel.

Es ist daher eine Aufgabe der Erfindung, eine Lebensmittelmatrix zu schaffen, welche derart zusammengesetzt ist, dass die Aktivität der eingesetzten probiotischen Bakterien im Lebensmittel eingeschränkt ist und somit die Lebensmittelmatrix durch den Zusatz probiotischer Bakterien nicht in einer für den Konsumenten des entsprechenden Lebensmittels negativen Weise verändert wird, aber der positive gesundheitliche Effekt nach Konsum des Lebensmittels im Menschen erhalten bleibt.

Es ist eine weitere Aufgabe der vorliegenden Erfindung, ein Verfahren zur Verfügung zu stellen, das die Herstellung einer solchen Lebensmittelmatrix ermöglicht. Insbesondere soll ein Verfahren zur Herstellung von trockenen fruchthaltigen Kompositionen ermöglicht werden, in der einerseits keine Beeinträchtigung bzw. Änderung in Geschmacks- oder Geruchsnote durch probiotische Bakterien stattfinden und andererseits durch die Anzahl an den probiotischen Bakterien während des gesamten Herstellungsprozesses und der Produktlagerung und -haltbarkeit immer noch ein gesundheitlicher positiver Effekt gemäß WHO Definition erreicht werden kann.

Zudem ist es eine Aufgabe der Erfindung, ein Produkt herzustellen, das zwar probiotische gesundheitsfördernde Eigenschaften aufweist, aber vom Konsumenten in ersten Linie als Fruchtprodukt wahrgenommen wird.

Gelöst wird die Aufgabe durch ein Verfahren zur Herstellung einer probiotischen Fruchtkomposition, das folgende Schritte umfasst:
(a) Mischen von
   (A) Keimen von einem probiotischen Mikroorganismus oder mehreren probiotischen Mikroorganismen und
   (B) einer Fruchtmasse hergestellt aus einer Frucht oder einer Mischung aus Früchten, wobei Frucht insbesondere aus der Gruppe ausgewählt ist, die Kernobst, Steinobst, Beerenobst und Gemüse umfasst, und wobei die Fruchtmasse (B) aus der Gruppe ausgewählt wird, die Fruchtstücke, Fruchtpüree, Fruchtpüreekonzentrat, Fruchtpulpe und deren Mischungen umfasst, und
   (C) optional Wasser, unter Erhalt einer Fruchtvormischung,
(b) Einfrieren der Fruchtvormischung,
(c1) Zerkleinern der gefrorenen Fruchtvormischung und/ oder optional Fraktionierung der erhaltenen Partikel nach gewünschter Größe,
(c) Gefriertrocknung der im Schritt (c1) hergestellten Partikel und/oder der in Schritt b) hergestellten eingefrorenen Fruchtvormischung unter Erhalt der probiotischen Fruchtkomposition.

Das Zerkleinern und/oder optionale Fraktionieren gemäß Schritt c1) kann gemäß der Erfindung vor und/oder nach Schritt c) der Gefriertrocknung durchgeführt werden.

Ein probiotischer Mikroorganismus (A) oder eine Mischung zumindest zweier probiotischer Mikroorganismen (A) können im Rahmen der Erfindung ausgewählt werden aus der Gruppe von Bakterien, welche Bakterien und Hefen der Gattungen *Lactobacillus, Bifidobacterium, Enterococcus, Lactococcus, Propionibacterium, Bacillus, Streptococcus, Escherichia,* Saccharomyces, bevorzugt *Lactobacillus, insbesondere Lactobacillus acidophilus,* und/oder *Bifidobacterium,* insbesondere *Bifidobacterium animalis subspecies lactis,* umfasst.

Die Mengen an probiotischen Mikroorganismen, die im erfindungsgemäßen Verfahren verwendet werden, betragen mindestens 10 mg / 100 g der trockenen Fruchtkomposition, bevorzugt 100 bis 1000 mg / 100 g der trockenen Fruchtkomposition, besonders bevorzugt 500 bis 800 mg / 100 g der trockenen Fruchtkomposition. Diese minimale Menge an probiotischen Mikroorganismen führt zur Herstellung einer Komposition, in der mindestens 10⁶ KBE pro Verzehreinheit erreicht werden. In Rahmen der vorliegenden Erfindung wird unter "Verzehreinheit" eine Menge eines Lebensmittelprodukts verstanden, die zumindest laut Ansicht des Inverkehrbringers beziehungsweise gemäß allgemeiner Lebenserfahrung typischerweise pro Verzehrsitzung aufgenommen wird und von ca. 1 g bis ca. 250 g, bevorzugt ca. 30 g bis 100 g, besonders bevorzugt ca. 50 g wiegt. Dieses Lebensmittelprodukt kann entweder die erfindungsgemäße probiotische Fruchtkomposition enthalten oder die erfindungsgemäße probiotische Fruchtkomposition darstellen. Beispiele für Produkte und zugehörige typische Verzehreinheiten sind eine Portion Müsli mit 50 g, ein Müsliriegel mit 30 bis 50 g, ein Snacking Bite / Ball mit 10 bis 15 g, eine Portion Schokolade mit 20 g und ein Sachet mit Fruchtpulver mit 2 bis 10 g.

Der Begriff "Frucht" wird hier entsprechend der Begriffsbestimmung in Anhang II der Richtlinie 2012/12/ EU des Europäischen Parlaments und des Rates vom 19. April 2012 zur Änderung der Richtlinie 2001/112/EG des Rates über Fruchtsäfte und bestimmte gleichartige Erzeugnisse für die menschliche Ernährung verwendet. Soweit nicht anders angegeben werden mit dem Begriff "Frucht" alle Früchte bezeichnet, einschließlich Früchte einjähriger Pflanzen wie Tomaten, die auch als Fruchtgemüse bezeichnet werden. Als "Obst" werden die in rohem Zustand essbaren Früchte mehrjähriger Bäume oder Sträucher bezeichnet. Als "Gemüse" werden alle einjährigen Pflanzen oder Teile von einjährigen Pflanzen bezeichnet, die roh oder verarbeitet der menschlichen Ernährung dienen. Soweit nicht anders angegeben, umfasst der Begriff "Gemüse" damit auch Früchte von einjährigen Pflanzen wie von Tomaten, die auch als "Fruchtgemüse" bezeichnet werden.

Im Schritt c) werden die Partikel aus dem Schritt c1) und/ oder die eingefrorene Fruchtvormischung aus Schritt b) bevorzugt unter Vakuum bis zu einer Restfeuchte von maximal 5 Gew.-% bezogen auf das Gesamtgewicht der Fruchtkomposition getrocknet.

Für die Erfindung können grundsätzlich alle Früchte verwendet werden, wobei eine Frucht oder eine Mischung aus zumindest zwei Früchten eingesetzt werden kann. Im Rahmen der Erfindung bezeichnet somit der Begriff "Frucht" sowohl Obst (wie Kernobst, Steinobst, Beerenobst) als auch Gemüse.

Ein besonders gutes Ergebnis in Lagerungstests wurde von Früchten gezeigt, die Carotinoide wie alpha-Carotin, beta-Carotin, gamma-Carotin, Capsaxanthin, Lycopin, Zeaxanthin und/oder Lutein enthalten und eine Gelbfärbung bis zu einer Orangefärbung des Fruchtfleisches aufweisen. Im Zusammenhang mit der Färbung mit einem Gelbanteil spielt der Aspekt eine Rolle, dass mit abnehmendem Gelbanteil die Menge an Anthocyanen zunimmt, welche sich negativ auf die Stabilität auswirken können. Früchte mit einem Gelbanteil ihrer Färbung werden in Rahmen der vorliegenden Erfindung als "gelbe" Früchte bzw.

Früchte "mit einem gelben Fruchtfleisch" bezeichnet. Beispiele für gelbe Früchte sind Mangos, Papayas, Pfirsiche, Aprikosen (Marillen), Äpfel, Bananen, Orangen, Birnen, Honigmelone, Karotten, Kürbisse, gelbe Zucchini, gelbe Paprikas, Fenchel, carotinoidhaltige Algen in Frage, die beispielsweise Astaxanthin enthalten, usw. sowie Mischungen der genannten Früchte. Die Zusammensetzung dieser Früchte hat sich in Versuchen weniger nachteilig gegenüber der Lebensfähigkeit von probiotischen Bakterien gezeigt, da sich bestimmte Polyphenole, insbesondere Anthocyane, negativ auf die Stabilität der Probiotika auswirken. Die genannten "gelben" Früchte haben einen geringeren Anthocyan-Gehalt.

Die Fruchtmasse (B) gemäß der vorliegenden Erfindung wird aus der Gruppe ausgewählt, welche Fruchtstücke, Fruchtpüree, Fruchtpüreekonzentrat, Fruchtpulpe und deren Mischungen, optional unter Zusatz mindestens einer dafür geeigneten Flüssigkeit, welche aus der Gruppe ausgewählt ist, die zum Beispiel Wasser, Saft, Saftkonzentrat, Fruchtsüßen, Pflanzenmilchbasen usw., umfasst.

Die erfindungsgemäß hergestellte Fruchtkomposition hat in einer bevorzugten Ausführungsform der Erfindung einen Fruchtanteil von mindestens 20 Gew.-%, bevorzugt von mindestens 30 Gew.-%, besonders bevorzugt von mindestens 35 Gew.-% bezogen auf die trockene Fruchtkomposition. Der maximale Fruchtgehalt in der erfindungsgemäßen Fruchtkomposition kann je nach Anwendungsfall beliebig hoch sein, solange die Fruchtkomposition noch einen wirksamen Anteil zumindest eines Probiotikums in ihrer Matrix aufweist. Beispielsweise kann der maximale Fruchtgehalt bei 99,99 Gew.-% liegen.

Der Fruchtanteil in der Fruchtkomposition wird gebildet von den Bestandteilen der eingesetzten Früchte ohne deren Stiele und Kerne, optional auch ohne deren Schale wie etwa im Fall von Zitrusfrüchten oder Melonen, wobei den eingesetzten Früchten lediglich Wasser entzogen wurde. Das Entziehen von Wasser erfolgt dabei im Rahmen des Trocknungsschrittes c). Der Fachmann versteht, dass der Entzug von Wasser das Entfernen weiterer Bestandteile, beispielsweise flüchtige Aromastoffe, der zumindest einen eingesetzten Frucht in einem gewissen Umfang mit sich bringen kann.

Im Rahmen des erfindungsgemäßen Verfahrens wird darauf geachtet, dass zumindest während der Durchführung der Schritte a), b), c1) und c) keine Fermentation erfolgt. Fermentierte Lebensmittel nicht für alle Menschen geeignet sind, insbesondere nicht für Histaminintolerante. Gerade die Fermentierung erhöht den Histamingehalt des Lebensmittels enorm, so dass sie bei entsprechend empfindlichen Menschen zu den typischen Symptomen einer Histaminintoleranz führen, wie z. B. Durchfall, Herzklopfen, Kurzatmigkeit, Hautrötungen, eine laufende Nase und geschwollene Augen.

Bei der Fermentation werden die im Lebensmittel enthaltenen Kohlenhydrate von den Bakterien verstoffwechselt. Dabei entstehen Milchsäure und Kohlendioxid und man würde Fruchtprodukte erhalten, deren Geschmack, Geruch und Farbe sich aufgrund der Fermentation deutlich von der unfermentierten Fruchtkomposition gemäß der Erfindung unterscheidet.

Im Rahmen der Erfindung liegt daher die Fruchtmatrix der Fruchtkomposition in einer unfermentierten Form gegenüber der zumindest einen Frucht vor, aus der die Fruchtmatrix hergestellt wurde. Die Erfindung ermöglicht es, ohne einen Fermentationsschritt ein Fruchtprodukt zu erhalten, das neben guten Lagerungseigenschaften und einem hohen Gehalt an probiotischen Mikroorganismen auch keinen Unterschied in Geschmack, Geruch und Farbe zu einer gleich gelagerten Fruchtkomposition ohne Probiotikum aufweist.

Im Schritt a) kann zusätzlich mindestens ein Gelier- und/oder Verdickungsmittel (C) zugesetzt werden, um insbesondere auch nach einem Gefrierschritt, für den eine erhöhte Viskosität des Gefriergutes die Verarbeitbarkeit erleichtern kann, eine trocknungs- bzw. granulierfähige Komposition zu erhalten. Diese Komponente (C) ist insbesondere aus der Gruppe ausgewählt, welche Agar, Alginate, Carragenane, Gellane, Xanthane, Gummies, Pektine, Stärken und Cellulosen und deren Derivate sowie Mischungen der genannten Gelier- und/oder Verdickungsmittel umfasst. Die Verwendung eines Gelier- und/oder Verdickungsmittels (C) ist nicht für alle Fruchtsorten und nicht zwingend notwendig.

Im Schritt a) kann zusätzlich mindestens ein Trägerstoff (D) zugesetzt werden, welcher beispielsweise aus der Gruppe ausgewählt ist, welche Glukosesirup (flüssig oder als Pulver), Zuckersirupe, Maltodextrin, Inulin, Pektin und faserreiche Fruchtpulpe sowie Mischungen davon umfasst. Der Zusatz mindestens eines Trägerstoffs erhöht die Trockenmasse und senkt damit den Wassergehalt und entsprechend den Trocknungsaufwand.

Für die Herstellung der Fruchtvormischung wird im Schritt a) die Komponente (A) mit der Mischung, die die Komponente (B) und optional Wasser enthält, bei Raumtemperatur, bevorzugt bei einer Temperatur im Bereich von 16°C bis 25 °C, innerhalb eines Zeitraums von 15 min bis 60 min, bevorzugt von 20 min bis 30 min gemischt. In einer bevorzugten Form der Erfindung werden die Fruchtmasse (B) und optional Wasser vorgelegt, anschließend Komponente (A) und ggf. die Komponente (D) hinzugegeben, wobei ein Gelier- oder Verdickungsmittel (C) als Letztes zugegeben werden kann.

Bei der Herstellung der erfindungsgemäßen Fruchtkomposition wird der zumindest eine probiotische Mikroorganismus (A) somit direkt in die Fruchtmasse (B) eingearbeitet. Optional kann dabei zunächst eine Vormischung aus Wasser und Keimen von einem probiotischen Mikroorganismus oder mehreren probiotischen Mikroorganismen hergestellt werden. Dies ist dann vorteilhaft, wenn die Probiotika in Pulverform zugesetzt werden, denn dann löst sich das Pulver erst einmal auf und es wird einer Klumpenbildung entgegen gewirkt. Eine solche Vormischung kann direkt in die Fruchtmasse eingearbeitet werden.

Die Fruchtmasse (B) mit dem darin homogen verteilten Keimen beziehungsweise koloniebildenden Einheiten (KBE) des zumindest einen probiotischen Mikroorganismus bildet nach Entzug von Wasser im Trocknungsschritt c) die Matrix der erfindungsgemäßen Fruchtkomposition unabhängig davon, in welcher Partikelgröße beziehungsweise ob sie zerkleinert oder unzerkleinert vorliegt. Die Matrix der erfindungsgemäßen Fruchtkomposition weist damit in ihrem Inneren keine Korngrenzen auf, wie sie etwa durch Verpressen einer Trockenfruchtkomponente gemischt mit Partikeln eines Probiotikums erzeugt werden würde. Die Matrix der erfindungsgemäßen Fruchtkomposition besteht pro betrachteter räumlichen Einheit - also pro Partikel oder pro untersuchtem Stück der gefriergetrockneten Fruchtvormischung vor dem Zerkleinern - aus einem durchgehenden Gerüst, das nach dem Entzug von Wasser durch das Gefriertrocknen c) aus der Fruchtvormischung zurückbleibt, in welchem probiotische Mikroorganismen eingelagert sind.

Im Schritt b) wird die Fruchtvormischung, bevorzugt unverpackt, eingefroren. Vor der Durchführung des Gefrierens in Schritt b) kann ein Schritt b1) durchgeführt werden, in welchem die Fruchtvormischung auf eine Temperatur unterhalb von 15°C vorgekühlt wird.

Als Gefrierverfahren können Kontaktgefrierverfahren (Plattenfroster), kryogene Gefrierverfahren oder Kaltluftgefrierverfahren angewendet werden, wobei das kryogene Gefrierverfahren bevorzugt wird. Das Gefrieren wird dabei mit Hilfe eines Gefriertunnels durchgeführt, wobei als Kältemittel z. B. Stickstoff oder Kohlendioxid oder Ähnliches verwendet werden können. Dabei wird die Fruchtvormischung innerhalb kurzer Zeit auf eine Kerntemperatur von mindestens -18 °C, bevorzugt weit unter -20°C, insbesondere auf einen Bereich von -95°C bis - 105°C, heruntergekühlt.

Nach dem Gefrierverfahren im Schritt b) wird die Fruchtvormischung in Form von Fruchtplatten erhalten. Diese gefrorene Fruchtvormischung kann in einer Ausführungsform der Erfindung im Schritt c1) in Partikel mit einer dafür geeigneten Vorrichtung auf eine gewünschten Größe, bevorzugt ca. 1 mm bis ca. 20 mm, besonders bevorzugt ca. 2 mm bis ca. 10 mm, ganz besonders bevorzugt ca. 1 mm bis ca. 3 mm gebrochen oder in einer Mühle vermahlen und ggf. nach Größe fraktioniert werden, z.B. durch Absieben. Die "Größe" der Partikel bezeichnet deren größte Ausdehnung. Durch Sieben werden bei einer den Grenzen des Bereichs für die "Größe" entsprechenden Maschenweite des Siebs mit der größeren und des Siebs mit der kleineren Maschenweite Bruchstücke mit einer "Größe" in dem betreffenden Größenbereich aus der Gesamtheit der Partikel heraussortiert (fraktioniert).

Die erhaltenen Partikel können je nach Zerkleinerungsverfahren unterschiedliche Formen aufweisen.

In einer bevorzugten Ausführungsform der Erfindung wird eine gefrorene Fruchtvormischung über eine Mühle mit einem Siebeinsatz mit gewünschter Siebgröße gebrochen. Danach können die erhaltenen Partikel wie oben erläutert mit Hilfe eines Obersiebes und/oder Untersiebes in die gewünschte Größe, bevorzugt im Bereich von 2 mm bis 10 mm oder im Bereich von 1 mm bis 3 mm groß, aufgeteilt werden.

Im Rahmen der Erfindung ist es auch möglich, die in Schritt b) erhaltenen Fruchtplatten der Gefriertrocknung c) zuzuführen und anschließend eine Zerkleinerung c1) der gefriergetrockneten Fruchtplatten durchzuführen. Auf diese Weise kann auch ein pulverförmiges Produkt hergestellt werden.

Im Schritt c) werden die Fruchtpartikel aus dem Schritt c1) oder die Fruchtplatten aus dem Schritt b) unter Vakuum in Trocknungskammern einer Gefriertrocknungsanlage bis zu einer Restfeuchte von < 5 Gew.-% bezogen auf das Gesamtgewicht der trockenen Fruchtkomposition getrocknet.

Der Druck in den Trocknungskammern der für Schritt c) verwendeten Gefriertrocknungsanlage wird insbesondere auf mindestens ca. 0,1 mbar, bevorzugt auf ca. 1,2 mbar, eingestellt. Gemäß einer Ausführungsform der Erfindung umfasst die Gefriertrocknung in Schritt c) eine Trocknungsphase bei Temperaturen im Bereich von 70 °C bis 120 °C, bevorzugt von 82°C bis 120°C, besonders bevorzugt 90 °C bis 120 °C, ganz besonders bevorzugt bei 115 °C. Die Temperatur der Radiatoren, welche sich in der Trocknungskammer befinden, beträgt dann zu Beginn ca. 70 °C bis ca. 120 °C, bevorzugt 82°C bis 120°C, besonders bevorzugt ca. 90 °C bis ca. 120 °C, ganz besonders bevorzugt ca. 115 °C, und senkt sich graduell auf ca. 30 °C bis ca. 50 °C, bevorzugt auf 40 °C, ab. Die Trocknungszeit beträgt ca. 10 bis ca. 48 h, bevorzugt ca. 10 bis ca. 24 h, besonders bevorzugt ca. 16 h.

Das nach dem erfindungsgemäßen Verfahren hergestellte Produkt stellt eine trockene probiotische Fruchtkomposition dar, die bevorzugt in Form eines Granulats vorliegt. Die Restfeuchte im Produkt beträgt maximal 5 Gew.-%, bevorzugt bis zu 3 Gew.-%, bezogen auf das Gesamtgewicht der trockenen Fruchtkomposition. Die bevorzugte Partikelgröße des Granulats liegt im Bereich von ca. 1 mm bis ca. 20 mm, bevorzugt im Bereich von ca. 2 mm bis ca. 10 mm, ganz besonders bevorzugt im Bereich von ca. 1 mm bis ca. 3 mm. Das erhaltene Granulat kann ggf. zu Pulver mit einer Partikelgröße im Bereich von ca. 10 um (Mikrometern) bis ca. 1000 um vermahlen werden. Ein entsprechendes Pulver kann beispielsweise in Sachets zur direkten Konsumption oder auch als Bestandteil einer Getränkepulverrezeptur verwendet werden.

Die nach dem erfindungsgemäßen Verfahren hergestellten probiotischen Fruchtkompositionen in Form von Granulaten oder Pulvern weisen keine Veränderung in Fruchtgeschmack und Farbe, die durch probiotische Mikroorganismen verursacht werden können, auf im Vergleich mit Granulaten oder Pulvern, die in entsprechender Weise, jedoch ohne Zusatz an Probiotika hergestellt werden.

Gegenstand der Erfindung ist auch eine probiotische Fruchtkomposition, die folgende Komponenten umfasst:
(A) Keime mindestens eines probiotischen Mikroorganismus' und
(B) eine Fruchtmatrix hergestellt aus zumindest einer Frucht oder einer Mischung aus Früchten, welche insbesondere aus der Gruppe ausgewählt ist, die Kernobst, Steinobst, Beerenobst und Gemüse sowie Mischungen der genannten Früchte umfasst, und hergestellt aus einer Fruchtmasse aus der Gruppe, die Fruchtstücke, Fruchtpüree, Fruchtpüreekonzentrat, Fruchtpulpe und deren Mischungen umfasst,

wobei die Fruchtkomposition in Form eines Granulats oder Pulvers vorliegt, in welchem der zumindest eine probiotische Mikroorganismus gleichmäßig verteilt vorliegt,
und wobei die Fruchtmatrix der Fruchtkomposition in einer unfermentierten Form gegenüber dem Fruchtfleisch der zumindest einen Frucht, aus welcher die Fruchtmatrix hergestellt wurde, vorliegt,
und welche mit dem erfindungsgemäßen Verfahren hergestellt worden ist.

Bevorzugt werden die oben bereits beschriebenen Früchte, die Carotinoide enthalten und eine Gelbfärbung des Fruchtfleisches aufweisen.

Die erfindungsgemäße Fruchtkomposition liegt in Form eines Granulats oder Pulvers vor. Der zumindest eine probiotische Mikroorganismus (A) ist wie oben beschrieben in die Fruchtmatrix aus der Fruchtmasse (B) der Fruchtkomposition direkt eingearbeitet. Anders gesagt ist der probiotische Mikroorganismus (A) in der Fruchtmatrix gleichmäßig verteilt.

Die Fruchtkomposition kann zusätzlich insbesondere in einer effektiven Menge mindestens ein Geliermittel und/oder zumindest ein Verdickungsmittel (C) enthalten, das bevorzugt aus der Gruppe ausgewählt wird, die Agar, Alginate, Carragenane, Gellane, Xanthane, Gummies, Pektine, Stärken und Cellulosen und deren Derivate sowie Mischungen davon umfasst.

Der Fruchtkomposition kann zusätzlich mindestens ein weiterer Trägerstoff (D) zugesetzt werden, der bevorzugt aus der Gruppe ausgewählt wird, die Glukosesirup (flüssig oder als Pulver), Zuckersirupe, Maltodextrin, Inulin, Pektine und faserreiche Fruchtpulpe sowie Mischungen davon umfasst.

Der Gehalt an probiotischen Keimen des zumindest einen probiotischen Mikroorganismus' (A) in der Fruchtkomposition beträgt in einer bevorzugten Ausführungsform der Erfindung mindestens 10⁶ KBE pro Verzehreinheit.

Für die Lagerung der erfindungsgemäßen probiotischen Fruchtkomposition sind alle für die Lagerung von probiotischen Produkten bekannten Bedingungen geeignet. Die Menge an lebensfähigen probiotischen Mikroorganismen in der erfindungsgemäßen probiotischen Fruchtkomposition oder einem Lebensmittel umfassend die erfindungsgemäße probiotische Fruchtkomposition kann insbesondere bis zu 18 Monate bei der Temperatur von -18 °C bis 20 °C, bevorzugt von 4 °C bis 8 °C, unverändert bleiben. Bei Raumtemperatur beträgt die Haltbarkeit einer erfindungsgemäßen probiotischen Fruchtkomposition oder eines Lebensmittels umfassend die erfindungsgemäße probiotische Fruchtkomposition mindestens sechs Monate. Auch keine Änderung der Sensorik, also weder in Geschmack noch in Geruch und Farbe, des Fruchtproduktes können festgestellt werden. Somit ermöglicht das erfindungsgemäße Verfahren beziehungsweise die erfindungsgemäßen probiotischen Fruchtkomposition oder ein Lebensmittel umfassend die erfindungsgemäße probiotische Fruchtkomposition eine Haltbarkeit von bis zu 18 Monaten der hergestellten Komposition.

Ein weiterer Gegenstand der Erfindung ist die Verwendung der erfindungsgemäßen Fruchtkomposition als Nahrungsmittel oder zur Herstellung von Lebensmittelprodukten, insbesondere trockenen Produkten oder Produkten mit einer niedrigen Wasseraktivitätswert (a_{w}), wie beispielsweise Müsli, Brei, Riegel, Schokolade usw. Insbesondere kann die erfindungsgemäße Fruchtkomposition verwendet werden in Lebensmitteln mit einer Wasseraktivität von maximal 0,8, bevorzugt mit einer Wasseraktivität von maximal 0,5, besonders bevorzugt mit einer Wasseraktivität von maximal 0,3. Ein Lebensmittelprodukt, das die erfindungsgemäßen Fruchtkomposition enthält, stellt auch einen Gegenstand der Erfindung dar. Der Gehalt der erfindungsgemäßen Fruchtkomposition im Lebensmittelprodukt beträgt ca. 0,1 Gew.-% bis ca. 99,9 Gew.-% bezogen auf das Gesamtgewicht des Lebensmittelprodukts oder Nahrungsergänzungsmittels.

Bei der Herstellung von Lebensmittelprodukten, die gemischte granulierte Medien aufweisen (wie z.B. Müsli oder Brei), ist die erfindungsgemäße Fruchtkomposition in Form von Granulat aufgrund der größeren Dimensionen der einzelnen Partikel besonders gut geeignet im Vergleich zum handelsüblichen probiotischen Pulver, da keine Entmischung (durch "Paranuss-Effekt") des Granulats von den weiteren Zutaten in einem gemischten Medium wie etwa einem Müsli nach mehrfachem Rütteln an einer Packung (z.B. beim Transport) auftritt. Durch die rüttelnde Bewegung entstehen kurzzeitig Hohlräume, in die bevorzugt die kleineren Bestandteile des Müslis (wie probiotische Pulver) rutschen. Somit kann durch Verwendung des erfindungsgemäßen Granulats die Verteilung der Probiotika im Lebensmittelprodukt verbessert und visualisiert werden.

Die Erfindung soll anhand der beigefügten Beispiele und der Figur erläutert werden, ohne jedoch auf die speziell beschriebene Ausführungsform beschränkt zu sein. Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

Es zeigt:

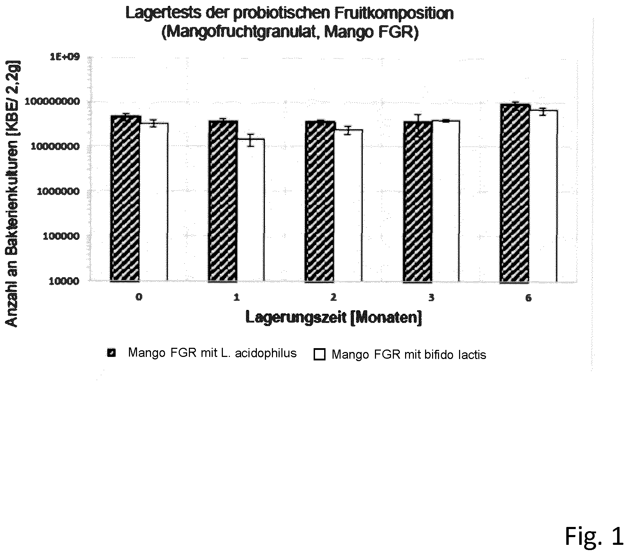

- Figur 1:: ein Diagramm der Ergebnisse von Lagertests von zwei probiotischen Fruchtkompositionen gemäß zwei Ausführungsformen der Erfindung.

### Ausführungsbeispiel 1:

### Mangofruchtgranulat (Mango FGR) mit Lactobacillus acidophilus

### 1. Mischen

Im Rahmen von Schritt a) des erfindungsgemäßen Verfahrens werden Glukosesirup, Mangopüreekonzentrat, Alginat und probiotisches Pulver von Lactobacillus acidophilus für 20 bis 30 min unter Zugabe von Wasser bei Raumtemperatur gemischt. Dazu werden Mangopüreekonzentrat und Wasser vorgelegt. Für die Rohware kann die Trockenmasse bestimmt werden, so dass der Fachmann die Menge an zugesetztem Wasser für die Zwecke des Verfahrens festlegen kann. Das Mangopüree besteht aus der kompletten Frucht ohne deren Schale und ohne Kern. Anschließend werden Glukosesirup und Probiotika hinzugegeben. Alginat wird zuletzt zugesetzt. In der folgenden Tabelle ist die Zusammensetzung der Mischung angegeben.

**Tabelle 1:**

| | |
|---|---|
| Glukosesirup | 20-22,5 Gew.-% |
| Mangopüreekonzentrat | 36-39 Gew.-% |
| Alginat E410 | 0,2-0,75 Gew.-% |
| Lactobacillus acidophilus | 0,01-1 Gew.-% |
| Wasser | 39 - 41% Gew.-% |

Alle Angaben in "Gew.-%" sind auf das Gesamtgewicht der entsprechenden Komposition bezogen.

### 2. Einfrieren

Die erhaltene Mischung wird im Rahmen des Schrittes b) des erfindungsgemäßen Verfahrens über einen Stickstofffroster eingefroren. Der Prozess ist kontinuierlich. Die Fruchtvormischung wird auf ein Band aufgetragen, dass einen Gefriertunnel durchläuft und kommt als gefrorene Platte heraus. Die Temperatur im Stickstofffroster (Produktion) ist deutlich unter -20 °C und kann insbesondere auf ca. -100°C, beispielweise im Bereich von -95°C bis -105°C, eingestellt werden.

### 3. Zerkleinern

Die gefrorene Platte wird bei der hier beschriebenen Ausführungsform der Erfindung in einem Schritt c1) über eine Mühle mit einem Siebeinsatz, der die Herstellung des Fruchtgranulats mit einer gewünschten Größe ermöglicht, gebrochen. Danach wird mit einem Ober- und/ oder Untersieb in die gewünschte Größe fraktioniert. In diesem Fall beträgt die Teilchengröße der Fruchtpartikel mindestens 2 mm und maximal 10 mm.

### 4. Trocknung

Zur Vorbereitung des Schrittes c) der Gefriertrocknung im Rahmen des erfindungsgemäßen Verfahrens werden die Fruchtpartikel in Schalen gefüllt und diese werden in Trocknungskammern gestellt. Für die Trocknung gemäß Schritt c) werden die Trocknungskammern der verwendeten Gefriertrocknungsanlage auf 1,2 mbar eingestellt. Die Temperatur der Radiatoren, welche sich in der Trocknungskammer befinden, beträgt zu Beginn 115 °C und senkt sich graduell auf 40 °C ab. Gesteuert wird dies über die Verdampfungsrate. Die Trocknungszeit beträgt 16 h.

### Ausführungsbeispiel 2:

### Mangofruchtgranulat (Mango FGR) mit Bifidobacterium lactis

Ebenso wie oben anhand des Ausführungsbeispiels 1 wurde ein Mangofruchtgranulat hergestellt, indem statt Keimen von *Lactobacillus acidophilus* Keime von *Bifidobacterium lactis* verwendet wurden.

### Ausführungsbeispiele 3 und 4:

### 100% Fruchtbasiertes Mangofruchtgranulat (Mango FGR 100) mit Bifidobacterium lactis oder Lactobacillus acidophilus

Ebenso wie oben anhand des Ausführungsbeispiels 1 und 2 wurde ein Mangofruchtgranulat hergestellt, indem der Mangofruchtanteil >99 Gew.% liegt, keine Verdickungsmittel und Glukosesirup verwendet werden und jeweils Keime von *Lactobacillus acidophilus* beziehungsweise Keime von *Bifidobacterium lactis* verwendet wurden.

Im Rahmen der Erfindung können gemäß den oben beschriebenen Ausführungsbeispielen auch Mischungen von Keimen, insbesondere Mischungen aus *Lactobacillus acidophilus* und *Bifidobacterium lactis,* verwendet werden.

An den derart hergestellten erfindungsgemäßen probiotischen Fruchtkompositionen aus Mangofruchtfleisch mit in der Fruchtmatrix eingebetteten Keimen von *Lactobacillus acidophilus* oder *Bifidobacterium lactis* wurden Lagertests durchgeführt.

### Durchführung der Nachweisbestimmung

Für jede Analyse wurde 2,2 g des probiotischen Fruchtgranulats gemäß Ausführungsbeispiel 1 oder gemäß Ausführungsbeispiel 2 abgewogen. Das Granulat wurde in einem Mörser zu feinem Pulver zerkleinert. Dieses Pulver wurde mit 100 ml destilliertem Wasser innerhalb von 30 min zu einer Vormischung vermischt. Die weiteren Schritte wurden in einer Sicherheitswerkbank (engl. "laminar airflow cabinet") durchgeführt. 1 ml der Vormischung wurde in 9 ml einer 0,9%igen NaCl-Lösung pipettiert. Dieser Verdünnungsschritt wurde drei Mal durchgeführt. Nach jeder Verdünnung wurde die Mischlösung 10 s mittels Reagenzglasmischgerät (Vortex) durchmischt. Die zwei letzten Verdünnungsschritte wurden auf MRS-Agar ausplattiert. Es wurden jeweils zwei Platten pro Verdünnungsstufe betrachtet. 100 µl der Lösung wurde auf eine auf einem Drehteller vorgelegte MRS-Agarplatte pipettiert. Der Drehteller wurde 10 s lang in Rotation betrieben, und die Flüssigkeit wurde mit einem Drigalskispatel gleichmäßig verteilt.

Die fertigen Agarplatten mit *L.acidophilus* wurden für 3 Tage bei 37 °C inkubiert. Die Agarplatten mit *B.lactis* wurden in einen Anaerobiertopf gestellt und für 3 Tage bei 37 °C unter einer anaeroben Atmosphäre bei einer relativen Feuchte im Bereich von 90 bis 96% bebrütet. Die anaerobe Atmosphäre wurde durch CO2-Generatoren in einem Anaerobiertopf bereitgestellt. Über einen Farbindikator im Topf wurde der Sauerstoffgehalt kontrolliert.

In Figur 1 sind zur Darstellung der Stabilitätsdaten Werte für die Anzahl koloniebildender Einheiten KBE aus 2,2 g der jeweiligen probiotischen Fruchtkomposition über der Lagerungszeit in Monaten aufgetragen. Es wurden Proben unmittelbar nach der Herstellung (Lagerungszeit 0 Monate) und nach einer Lagerungszeit von einem, zwei, drei und sechs Monaten untersucht. Es wurden wie oben beschrieben zwei Verdünnungsstufen mit jeweils 2 Platten pro Verdünnungsstufe betrachtet. Aus den daraus ermittelten 4 Messwerten wurde ein Mittelwert generiert. Alle ausgewerteten Platten hatten mehr als 10 und weniger als 300 Kolonien. Der anhand der dunkel schraffierten Balken dargestellte Mittelwert bezieht sich auf die Proben des Mangofruchtgranulats mit *L. acidophilus.* Der anhand der unausgefüllten Balken dargestellte Mittelwert bezieht sich auf die Proben des Mangofruchtgranulats mit *B.lactis.* Die Daten für die Mittelwerte sind in der folgenden Tabelle zusammengestellt.

**Tabelle 2: Durchschnittswerte (KBE/ 2,2g)**

| Probiotische Fruchtkomposition | Lagerungszeit (Monate) | | | | |
|---|---|---|---|---|---|
| | 0 | 1 | 2 | 3 | 6 |
| Mango FGR mit *L. acidophilus* | 4,56 . 10⁷ | 3,63 · 10⁷ | 3,48 · 10⁷ | 3,50 · 10⁷ | 8,70 · 10⁷ |
| Mango FGR mit *Bifido lactis* | 3,35 . 10⁷ | 1,48 . 10⁷ | 2,40 . 10⁷ | 3,96 · 10⁷ | 6,65 . 10⁷ |

Neben guten Lagerungseigenschaften und einem hohen Gehalt an probiotischen Mikroorganismen (s. die Tabellen oben) weist die erfindungsgemäße Fruchtkomposition keinen Unterschied in Geschmack, Geruch und Farbe zu einer gleich gelagerten Fruchtkomposition ohne Probiotikum auf.

Es ist dem Fachmann ersichtlich, dass die Erfindung nicht auf die vorstehend beschriebenen Beispiele beschränkt ist, sondern vielmehr in vielfältiger Weise variiert werden kann. Insbesondere können die Merkmale der einzeln dargestellten Beispiele auch miteinander kombiniert oder gegeneinander ausgetauscht werden.

## Patentansprüche

1. Verfahren zur Herstellung einer probiotischen Fruchtkomposition, das folgende Schritte umfasst:
a) Mischen von
(A) Keimen von einem probiotischen Mikroorganismus oder mehreren probiotischen Mikroorganismen und
(B) einer Fruchtmasse hergestellt aus einer Frucht oder einer Mischung aus Früchten, wobei Frucht insbesondere aus der Gruppe ausgewählt ist, die Kernobst, Steinobst, Beerenobst und Gemüse umfasst, und wobei die Fruchtmasse (B) aus der Gruppe ausgewählt wird, die Fruchtstücke, Fruchtpüree, Fruchtpüreekonzentrat, Fruchtpulpe und deren Mischungen umfasst, und
(C) optional Wasser,
unter Erhalt einer Fruchtvormischung,
b) Einfrieren der Fruchtvormischung unter Erhalt einer gefrorenen Fruchtvormischung,
c1) Zerkleinern der gefrorenen Fruchtvormischung und/oder optional Fraktionierung der erhaltenen Partikel nach gewünschter Größe, und
c) Gefriertrocknung der im Schritt c1) hergestellten Partikel und/oder der in Schritt b) hergestellten eingefrorenen Fruchtvormischung unter Erhalt der probiotischen Fruchtkomposition,
wobei der Schritt c1) vor und/oder nach Schritt c) durchgeführt werden kann.

2. Verfahren nach Anspruch 1,
**dadurch gekennzeichnet, dass**
im Schritt c) die Partikel aus dem Schritt c1) und/oder die gefrorene Fruchtvormischung aus Schritt b) unter Vakuum bis zu einer Restfeuchte von maximal 5 Gew.-% bezogen auf das Gesamtgewicht der Fruchtkomposition getrocknet werden.

3. Verfahren nach Anspruch 1 oder 2,
**dadurch gekennzeichnet, dass**
als Frucht zumindest eine Frucht eingesetzt wird, welche gelbes Fruchtfleisch aufweist, wobei insbesondere zumindest eine Frucht eingesetzt wird, welche aus der Gruppe ausgewählt wird, die Mangos, Papayas, Pfirsiche, Aprikosen (Marillen), Äpfel, Bananen, Orangen, Birnen, Honigmelone, Karotten, Kürbisse, gelbe Zucchini, gelbe Paprikas und Fenchel, carotinoidhaltige Algen sowie Mischungen der genannten Früchte umfasst.

4. Verfahren nach mindestens einem der vorstehenden Ansprüche, **dadurch gekennzeichnet, dass**
die Fruchtmasse (B) aus der Gruppe ausgewählt wird, die Fruchtstücke, Fruchtpüree, Fruchtpüreekonzentrat, Fruchtpulpe und deren Mischungen, unter Zusatz mindestens einer dafür geeigneten Flüssigkeit, welche aus der Gruppe ausgewählt ist, die Wasser, Saft, Saftkonzentrat, Fruchtsüßen und Pflanzenmilchbasen umfasst.

5. Verfahren nach einem der vorstehenden Ansprüche,
**dadurch gekennzeichnet, dass**
zumindest während der Durchführung der Schritte a), b), c1) und c) keine Fermentation erfolgt.

6. Verfahren nach mindestens einem der vorstehenden Ansprüche, **dadurch gekennzeichnet, dass**
im Schritt a) zusätzlich mindestens ein Geliermittel und/oder zumindest ein Verdickungsmittel (C) zugesetzt wird, wobei diese Komponente (C) bevorzugt aus der Gruppe ausgewählt wird, die Agar, Alginate, Carragenane, Gellane, Xanthane, Gummies, Pektine, Stärken, Cellulosen und deren Derivate sowie Mischungen der genannten Gelier- und/oder Verdickungsmittel umfasst.

7. Verfahren nach mindestens einem der vorstehenden Ansprüche, **dadurch gekennzeichnet, dass**
im Schritt a) zusätzlich mindestens ein Trägerstoff (D) zugesetzt wird, wobei die Komponente (D) bevorzugt aus der Gruppe ausgewählt ist, die flüssigen Glukosesirup, Glukosesirup als Pulver, Zuckersirupe, Maltodextrin, Inulin, Pektin, faserreiche Fruchtpulpe sowie Mischungen davon umfasst.

8. Verfahren nach mindestens einem der vorstehenden Ansprüche, **dadurch gekennzeichnet, dass**
die Gefriertrocknung in Schritt c) eine Trocknungsphase bei Temperaturen im Bereich von 70 °C bis 120 °C, bevorzugt von 82°C bis 120°C, besonders bevorzugt 90 °C bis 120 °C, ganz besonders bevorzugt bei 115 °C umfasst.

9. Verfahren nach mindestens einem der vorstehenden Ansprüche, **dadurch gekennzeichnet, dass**
die erhaltene probiotischen Fruchtkomposition in Form eines Granulats bereitgestellt wird und optional zu Pulver vermahlen werden kann.

10. Probiotische Fruchtkomposition, die folgende Komponenten umfasst:
Keime mindestens eines probiotischen Mikroorganismus' und
eine Fruchtmatrix hergestellt aus zumindest einer Frucht oder einer Mischung aus Früchten, welche insbesondere aus der Gruppe ausgewählt ist, die Kernobst, Steinobst, Beerenobst und Gemüse sowie Mischungen der genannten Früchte umfasst, und hergestellt aus einer Fruchtmasse aus der Gruppe, die Fruchtstücke, Fruchtpüree, Fruchtpüreekonzentrat, Fruchtpulpe und deren Mischungen umfasst,
wobei die Fruchtkomposition in Form eines Granulats oder Pulvers vorliegt, in welchem der zumindest eine probiotische Mikroorganismus gleichmäßig verteilt vorliegt,
und wobei die Fruchtmatrix der Fruchtkomposition in einer unfermentierten Form gegenüber dem Fruchtfleisch der zumindest einen Frucht, aus welcher die Fruchtmatrix hergestellt wurde, vorliegt, und
welche mit einem Verfahren nach mindestens einem der Ansprüche 1 bis 9 hergestellt worden ist.

11. Fruchtkomposition nach Anspruch 10,
**dadurch gekennzeichnet, dass**
die Fruchtmatrix zumindest Fruchtfleisch einer Frucht umfasst, welche gelbes Fruchtfleisch aufweist und insbesondere aus der Gruppe ausgewählt ist, welche Mangos, Papayas, Pfirsiche, Aprikosen, Äpfel, Bananen, Orangen, Birnen, Honigmelone, Karotten, Kürbisse, gelbe Zucchini, gelbe Paprikas, Fenchel und carotinoidhaltige Algen sowie Mischungen der genannten Früchte umfasst.

12. Fruchtkomposition nach Anspruch 10 oder 11,
**dadurch gekennzeichnet, dass**
diese zusätzlich mindestens ein Geliermittel und/ oder ein Verdickungsmittel (C) enthält, wobei diese Komponente (C) bevorzugt aus der Gruppe ausgewählt wird, die Alginate, Pektine, Stärken, Cellulosen sowie Mischungen davon umfasst.

13. Fruchtkomposition nach Anspruch 10 bis 12,
**dadurch gekennzeichnet, dass**
diese zusätzlich mindestens ein weiterer Trägerstoff (D) zugesetzt wird, wobei diese Komponente (D) bevorzugt aus der Gruppe ausgewählt wird, die flüssigen Glukosesirup, Glukosesirup als Pulver, Zuckersirupe, Maltodextrin, Inulin, Pektin und faserreiche Fruchtpulpe sowie Mischungen davon umfasst.

14. Fruchtkomposition nach mindestens einem der Ansprüche 10 bis 13,
**dadurch gekennzeichnet, dass**
diese einen Gehalt an probiotischen Keimen des zumindest einen probiotischen Mikroorganismus' (A) in der Fruchtkomposition von mindestens 10⁶ KBE pro Verzehreinheit enthält.

15. Fruchtkomposition nach mindestens einem der Ansprüche 10 bis 14,
**dadurch gekennzeichnet, dass**
diese einen Fruchtanteil von mindestens 20 Gew.-%, bevorzugt von mindestens 30 Gew.-%, besonders bevorzugt von mindestens 35 Gew.-% bezogen auf die trockene Fruchtkomposition, insbesondere bezogen auf die trockene Fruchtkomposition mit einer Restfeuchte von maximal 5 Gew.-% bezogen auf das Gesamtgewicht der Fruchtkomposition, aufweist.

16. Verwendung einer Fruchtkomposition nach mindestens einem der Ansprüche 10 bis 15
als Nahrungsmittel oder zur Herstellung von Lebensmittelprodukten, insbesondere trockenen Produkten und/oder Produkten mit einem niedrigen Wert der Wasseraktivitätswert mit einem a_{w}-Wert von maximal 0,8, wie Müsli, Brei, Riegel und Schokolade.

## Claims

1. A method for producing a probiotic fruit composition, which comprises the following steps:
(a) mixing of
(A) bacteria of a probiotic microorganism or several probiotic microorganisms and
(B) a fruit mass produced from a fruit or a mixture of fruit, wherein fruit is in particular selected from the group, which comprises pome fruit, stone fruit, soft fruit, and vegetables, and wherein the fruit mass (B) is selected from the group, which comprises pieces of fruit, fruit puree, fruit puree concentrate, fruit pulp, and the mixtures thereof, and
(C) optionally water,
by obtaining a fruit premixture,
b) freezing the fruit premixture by obtaining a frozen fruit premixture,
c1) comminuting the frozen fruit premixture and/or optionally fractioning the obtained particles according to desired size, and
c) freeze-drying the particles produced in step c1) and/or the frozen fruit premixture produced in step b) by obtaining the probiotic fruit composition,
wherein step c1) can be performed prior to and/or after step c).

2. The method according to claim 1,
**characterized in that**
in step c), the particles from step c1) and/or the frozen fruit premixture from step b) are dried under vacuum up to a residual moisture of maximally 5% by weight, based on the total weight of the fruit composition.

3. The method according to claim 1 or 2,
**characterized in that**
at least one fruit is used as fruit, which has yellow fruit flesh, wherein in particular at least one fruit is used, which is selected from the group, which comprises mangoes, papayas, peaches, apricots, apples, bananas, oranges, pears, honeydew melon, carrots, squash, yellow zucchinis, yellow peppers and fennel, carotenoid-containing algae, as well as mixtures of the mentioned fruit.

4. The method according to at least any one of the preceding claims, **characterized in that**
the fruit mass (B) is selected from the group, which comprises pieces of fruit, fruit puree, fruit puree concentrate, fruit pulp and the mixtures thereof, by adding at least one liquid, which is suitable for this purpose, which is selected from the group, which comprises water, juice, juice concentrate, fruit sweeteners, and plant milk bases.

5. The method according to any one of the preceding claims, **characterized in that**
no fermentation takes place at least while performing the steps a), b), c1), and c).

6. The method according to at least any one of the preceding claims,
**characterized in that**
in step a), at least one gelling and/or at least one thickening agent (C) is additionally added, wherein this component (C) is preferably selected from the group, which comprises agar, alginates, carrageenans, gellans, xanthans, gums, pectins, starches, celluloses and the derivatives thereof, as well as mixtures of the mentioned gelling and/or thickening agents.

7. The method according to at least any one of the preceding claims,
**characterized in that**
in step a), at least one carrier substance (D) is additionally added, wherein the component (D) is preferably selected from the group, which comprises liquid glucose syrup, glucose syrup as powder, sugar syrups, maltodextrin, inulin, pectin, high-fiber fruit pulp, as well as mixtures thereof.

8. The method according to at least any one of the preceding claims,
**characterized in that**
the freeze-drying in step c) comprises a drying phase at temperatures in the range of 70°C to 120°C, preferably of 82°C to 120°C, particularly preferably 90°C to 120°C, most preferably at 115°C.

9. The method according to at least any one of the preceding claims,
**characterized in that**
the obtained probiotic fruit composition is provided in the form of a granulate and can optionally be ground into powder.

10. A probiotic fruit composition, which comprises the following components:
bacteria of at least one probiotic microorganism and
a fruit matrix produced from at least one fruit or a mixture of fruit, which is in particular selected from the group, which comprises pome fruit, stone fruit, soft fruit, and vegetables, as well as mixtures of the mentioned fruit, and produced from a fruit mass from the group, which comprises pieces of fruit, fruit puree, fruit puree concentrate, fruit pulp, and the mixtures thereof,
wherein the fruit composition is present in the form of a granulate or powder, in which the at least one probiotic microorganisms is present in an evenly distributed manner,
and wherein the fruit matrix of the fruit composition is present in an unfermented form compared to the fruit flesh of the at least one fruit, from which the fruit matrix was produced, and
which has been produced by means of a method according to at least any one of claims 1 to 9.

11. The fruit composition according to claim 10, **characterized in that**
the fruit matrix comprises at least fruit flesh of a fruit, which has yellow fruit flesh and is in particular selected from the group, which comprises mangoes, papayas, peaches, apricots, apples, bananas, oranges, pears, honeydew melon, carrots, squash, yellow zucchinis, yellow peppers, fennel, and carotenoid-containing algae, as well as mixtures of the mentioned fruit.

12. The fruit composition according to claim 10 or 11, **characterized in that**
it additionally has at least one gelling agent and/or one thickening agent (C), wherein this component (C) is preferably selected from the group, which comprises alginates, pectins, starches, celluloses, as well as mixtures thereof.

13. The fruit composition according to claim 10 to 12, **characterized in that**
at least one further carrier substance (D) is added thereto, wherein this component (D) is preferably selected from the group, which comprises liquid glucose syrup, glucose syrup as powder, sugar syrups, maltodextrin, inulin, pectin, and high-fiber fruit pulp, as well as mixtures thereof.

14. The fruit composition according to at least any one of claims 10 to 13,
**characterized in that**
it contains a content of probiotic bacteria of the at least one probiotic microorganism (A) in the fruit composition of at least 10⁶ CFU per unit of consumption.

15. The fruit composition according to at least any one of claims 10 to 14,
**characterized in that**
it has a fruit content of at least 20% by weight, preferably of at least 30% by weight, particularly preferably of at least 35% by weight, based on the dry fruit composition, in particular based on the dry fruit composition with a residual moisture of maximally 5% by weight, based on the total weight of the fruit composition.

16. Use of a fruit composition according to at least any one of claims 10 to 15
as food or for the production of food products, in particular dry products and/or products with a low value of the water activity value with an a_{w} value of maximally 0.8, such as cereal, porridge, bars, and chocolate.

## Revendications

1. Procédé de préparation d'une composition de fruits probiotiques, comprenant les étapes suivantes :
a) mélange
(A) de germes d'un micro-organisme probiotique ou de plusieurs micro-organismes probiotiques et
(B) d'une pâte de fruits préparée à partir d'un fruit ou d'un mélange de fruits, le fruit étant notamment choisi dans le groupe comprenant les fruits à pépins, les fruits à noyau, les fruits rouges et les légumes, et la pâte de fruits (B) étant choisie dans le groupe comprenant les morceaux de fruits, la purée de fruits, le concentré de purée de fruits, la pulpe de fruits et leurs mélanges, et
(C) d'eau facultativement,
pour obtenir un prémélange de fruits,
b) congélation du prémélange de fruits pour obtenir un prémélange de fruits congelé,
c) broyage du prémélange de fruits congelé et/ou, éventuellement, fractionnement des particules obtenues selon la taille souhaitée, et
d) lyophilisation des particules produites à l'étape c1) et/ou du prémélange de fruits congelé produit à l'étape b), pour obtenir une composition de fruits probiotiques, l'étape c1) pouvant être réalisée avant et/ou après l'étape c) .

2. Procédé selon la revendication 1,
**caractérisé en ce que**
à l'étape c), les particules de l'étape c1) et/ou le prémélange de fruits congelé de l'étape b) sont séchés sous vide jusqu'à une humidité résiduelle maximale de 5 % en poids par rapport au poids total de la composition de fruits.

3. Procédé selon la revendication 1 ou 2,
**caractérisé en ce que**
le fruit utilisé est au moins un fruit qui présente une pulpe jaune, en particulier au moins un fruit, qui est choisi dans le groupe comprenant les mangues, les papayes, les pêches, les abricots, les pommes, les bananes, les oranges, les poires, le melon miel, les carottes, les courges, les courgettes jaunes, les poivrons jaunes et le fenouil, les algues contenant des caroténoïdes ainsi que des mélanges des fruits mentionnés, étant utilisé.

4. Procédé selon au moins l'une des revendications précédentes,
**caractérisé en ce que**
la pâte de fruits (B) est choisie dans le groupe comprenant les morceaux de fruits, la purée de fruits, le concentré de purée de fruits, la pulpe de fruits et leurs mélanges, avec l'ajout d'au moins un liquide approprié à cet effet, qui est choisi dans le groupe comprenant l'eau, le jus, le concentré de jus, les sucres de fruits et les bases de lait végétal.

5. Procédé selon au moins l'une des revendications précédentes,
**caractérisé en ce que**
aucune fermentation n'a lieu au moins pendant la réalisation des étapes a), b), c1) et c).

6. Procédé selon au moins l'une des revendications précédentes,
**caractérisé en ce que**
à l'étape a), au moins un agent gélifiant et/ou au moins un agent épaississant (C) est en outre ajouté, ce composant (C) étant préférablement choisi dans le groupe comprenant l'agar, les alginates, les carraghénanes, les gellanes, les xanthanes, les gommes, les pectines, les amidons, les celluloses et leurs dérivés ainsi que des mélanges des agents gélifiants et/ou épaississants mentionnés.

7. Procédé selon au moins l'une des revendications précédentes,
**caractérisé en ce que**
à l'étape a), au moins un support (D) est en outre ajouté, le composant (D) étant préférablement choisi dans le groupe comprenant le sirop de glucose liquide, le sirop de glucose sous forme de poudre, les sirops de sucre, la maltodextrine, l'inuline, la pectine, la pulpe de fruits riche en fibres ainsi que des mélanges de ceux-ci.

8. Procédé selon au moins l'une des revendications précédentes,
**caractérisé en ce que**
la lyophilisation à l'étape c) comprend une phase de séchage à des températures comprises entre 70°C et 120°C, préférablement entre 82°C et 120°C, plus préférablement entre 90°C et 120°C, plus particulièrement préférablement à 115°C.

9. Procédé selon au moins l'une des revendications précédentes,
**caractérisé en ce que**
la composition de fruits probiotiques obtenue est mise à disposition sous forme de granulés et peut éventuellement être réduite en poudre.

10. Composition de fruits probiotiques, comprenant les composants suivants :
des germes d'au moins un micro-organisme probiotique et
une matrice de fruits préparée à partir d'au moins un fruit ou d'un mélange de fruits, qui est notamment choisi dans le groupe comprenant les fruits à pépins, les fruits à noyau, les fruits rouges et les légumes ainsi que les mélanges des fruits mentionnés, et préparée à partir d'une pâte de fruits choisie dans le groupe comprenant les morceaux de fruits, la purée de fruits, le concentré de purée de fruits, la pulpe de fruits et leurs mélanges,
la composition de fruits se présentant sous la forme de granulés ou de poudre, dans lesquels l'au moins un micro-organisme probiotique est réparti uniformément,
et dans lesquels la matrice de fruits de la composition de fruits se présente sous une forme non fermentée par rapport à la pulpe de l'au moins un fruit à partir duquel la matrice de fruits a été préparée, et
laquelle a été préparée par un procédé selon au moins l'une des revendications 1 à 9.

11. Composition de fruits selon la revendication 10, **caractérisée en ce que**
la matrice de fruits comprend au moins la pulpe d'un fruit qui présente une pulpe jaune et qui est notamment choisi dans le groupe comprenant les mangues, les papayes, les pêches, les abricots, les pommes, les bananes, les oranges, les poires, le melon miel, les carottes, les courges, les courgettes jaunes, les poivrons jaunes, le fenouil et les algues contenant des caroténoïdes ainsi que des mélanges des fruits mentionnés.

12. Composition de fruits selon la revendication 10 ou 11, **caractérisée en ce que**
celle-ci contient en outre au moins un agent gélifiant et/ou un agent épaississant (C), ce composant (C) étant préférablement choisi dans le groupe comprenant les alginates, les pectines, les amidons, les celluloses ainsi que des mélanges de ceux-ci.

13. Composition de fruits selon la revendication 10 à 12, **caractérisée en ce que**
au moins un autre support (D) est en outre ajouté, ce composant (D) étant préférablement choisi dans le groupe comprenant le sirop de glucose liquide, le sirop de glucose en poudre, les sirops de sucre, la maltodextrine, l'inuline, la pectine et la pulpe de fruits riche en fibres ainsi que leurs mélanges.

14. Composition de fruits selon au moins l'une des revendications 10 à 13,
**caractérisée en ce que**
celle-ci contient une teneur en germes probiotiques d'au moins un micro-organisme probiotique (A) dans la composition de fruits d'au moins 10⁶ UFC par unité de consommation.

15. Composition de fruits selon au moins l'une des revendications 10 à 14,
**caractérisée en ce que**
celle-ci présente une teneur en fruits d'au moins 20 % en poids, préférablement d'au moins 30 % en poids, plus préférablement d'au moins 35 % en poids par rapport à la composition de fruits sèche, en particulier par rapport à la composition de fruits sèche avec une humidité résiduelle maximale de 5 % en poids par rapport au poids total de la composition de fruits.

16. Utilisation d'une composition de fruits selon au moins l'une des revendications 10 à 15,
comme aliment ou pour la fabrication de produits alimentaires, en particulier de produits secs et/ou de produits ayant une faible valeur d'activité de l'eau avec une valeur a_{w} inférieure ou égale à 0,8, tels que le muesli, les bouillies, les barres et le chocolat.
